# EUROPÄISCHE PATENTSCHRIFT

(11) **EP 3 038 558 B1**
(45) Veröffentlichungstag und Bekanntmachung des Hinweises auf die Patenterteilung: **18.12.2019**
(21) Anmeldenummer: 14771208.7
(22) Anmeldetag: 29.08.2014
(51) Int. Cl.: A61B 90/00, A61B 34/20, A61B 5/06, A61B 17/00

(54) **VERFAHREN UND VORRICHTUNG ZUM NAVIGIEREN VON AKTIVEN CHIRURGISCHEN INSTRUMENTEN**
METHOD AND DEVICE FOR NAVIGATING ACTIVE SURGICAL INSTRUMENTS
PROCÉDÉ ET DISPOSITIF DE GUIDAGE D'INSTRUMENTS CHIRURGICAUX ACTIFS

(30) Priorität: 30.08.2013 DE 102013217328
(43) Veröffentlichungstag der Anmeldung: 06.07.2016
(73) Patentinhaber: Fiagon AG Medical Technologies, 16761 Hennigsdorf (DE)
(72) Erfinder: MUCHA, Dirk, 13467 Berlin (DE)
(74) Vertreter: Eisenführ Speiser
(86) Internationale Anmeldenummer: PCT/EP2014/068447
(87) Internationale Veröffentlichungsnummer: WO 2015/028646

(56) Entgegenhaltungen:
- WO-A1-2012/109760
- WO-A2-2013/010138
- DE-A1-102004 058 272
- US-A1- 2005 288 576
- US-A1- 2008 287 802
- US-A1- 2012 007 747

## Beschreibung

Die Erfindung betrifft ein Verfahren zum Erfassen von Position und Betriebszustand eines aktiven chirurgischen Instruments in einem Arbeitsbereich mittels eines Sensors, wobei das aktive chirurgische Instrument in einem eingeschalteten Betriebszustand mindestens ein elektromagnetisches Feld emittiert und mit dem Sensor das elektromagnetische Feld erfasst wird. Ferner betrifft die Erfindung ein medizinisches System zum Lokalisieren von aktiven chirurgischen Instrumenten in einem Arbeitsbereich, mit mindestens einem aktiven chirurgischen Instrument das ausgebildet ist, im aktiven Betriebszustand ein elektromagnetisches Feld zu emittieren.

Bei invasiven chirurgischen Eingriffen werden chirurgische Instrumente während einer Operation von einem Operateur in einem Operationsgebiet im Inneren eines Patienten bewegt. Als Operationsgebiet wird der Raum im Inneren des Patienten bezeichnet, der während der Operation potenziell oder effektiv von den eingesetzten chirurgischen Instrumenten beeinträchtigt wird. Zur Operation zählen das Ein- und Ausführen von chirurgischen Instrumenten in den Körper des Patienten hinein bzw. aus dem Körper des Patienten heraus sowie das Bewegen der chirurgischen Instrumente im Körper des Patienten und der Einsatz der chirurgischen Instrumente in einem im Operationsgebiet angeordneten und genau vorbestimmten Eingriffsgebiet. Als Eingriffsgebiet wird der Teil im Operationsgebiet bezeichnet, der vom Operateur zu bearbeiten ist. Hierbei kann es sich z.B. um zu entfernendes Gewebe oder zu verschließende Gefäße handeln. Demnach kann ein Operationsgebiet eine Vielzahl von Eingriffsgebieten umfassen. Neben den Eingriffsgebieten liegt im Operationsgebiet eine Vielzahl von sensiblen Strukturen, die vor Beschädigungen durch die chirurgischen Instrumente zu bewahren sind. Zu den sensiblen Strukturen zählen z.B. Gefäße, Organe, Nerven, Muskeln, Bänder, Sehnen und sonstiges, generell intaktes Gewebe, das erhalten bleiben soll, um die Auswirkungen der Operation auf ein notwendiges Minimum zu beschränken, da jede weitere Beeinträchtigung des Körpers des Patienten während der Operation das Gesundheitsrisiko des Patienten erhöhen und das Operationsergebnis negativ beeinflussen kann.

Bei der Durchführung invasiver chirurgischer Eingriffe kommen daher regelmäßig Lageerfassungssysteme zum Einsatz. Lageerfassungssysteme unterstützen den Operateur bei der Ermittlung der Lage - also der Position und Ausrichtung - chirurgischer Instrumente im Operationsgebiet. Bekannte Lageerfassungssysteme dienen insbesondere der Verbesserung des Navigierens des chirurgischen Instruments in ein Eingriffsgebiet im Körper des Patienten hinein, des Navigierens des chirurgischen Instruments während der Durchführung einer operativen Maßnahme sowie des Navigierens des chirurgischen Instruments aus dem Körper des Patienten heraus. Hierdurch können das Ergebnis sowie die Effizienz der durchzuführenden Operation verbessert werden. Darüber hinaus kann eine präzise Navigation des chirurgischen Instruments das Risiko einer unbeabsichtigten Beeinträchtigung oder Beschädigung von umliegendem Gewebe oder potenziell gefährdeter Nervenbahnen im Operationsgebiet reduzieren.

Üblicherweise erfassen Lageerfassungssysteme während der Operation die Position und Ausrichtung mindestens eines medizinischen z.B. chirurgischen Instruments und transformieren die erfassten Positionskoordinaten so dass Position und Ausrichtung des Instruments in z.B. Bilder des Patienten und insbesondere ggf. räumliche Darstellungen des Eingriffsgebietes eingeblendet werden können. In vielen Lageerfassungssystemen können Lageinformationen einer Vielzahl von unterschiedlichen chirurgischen Instrumenten ermittelt werden. Die erfassten Lageinformationen werden meistens zusammen mit präoperativ gewonnenen Planungsdaten und/oder intraoperativ gewonnenen Bilddaten auf einem Monitor visualisiert. Hierfür werden an bestimmbaren Stellen des Patienten sowie der chirurgischen Instrumente Sensoren (die auch als Lokalisatoren bezeichnet werden) angeordnet, deren Ausgangssignale als Lageinformationen im Operationsgebiet von einer Auswerteeinheit des Lageerfassungssystems bestimmbar sind.

Es ist beispielsweise bekannt, unterschiedliche medizinische Instrumente, wie z.B. Zeigeinstrumente, Sauger, Zangen, Nadeln, Skalpelle, Elektrotome, Kauter, u. ä. mit Lokalisatoren (Instrumentensensoren) zur Ermittlung von Lageinformationen für ein solches Lageerfassungssystem zu versehen und das jeweilige medizinische Instrument in dem Lageerfassungssystem zu registrieren. Bei der Registrierung wird die Lage eines Bezugspunktes - üblicherweise des Arbeitspunkts der Instrumentenspitze - mittels eines Lokalisators (Positionssensors) am Instrument relativ zu einem bekannten Punkt am Patienten (oder einem Objekt) eingemessen und an das Lageerfassungssystem übermittelt. Auf diese Weise kann die vom Lageerfassungssystem bestimmte Lage des Bezugspunktes und Ausrichtung des medizinischen Instruments mit bekannten Punkten am Patienten oder einem Objekt in Beziehung gesetzt werden um anschließend die vom Lageerfassungssystem erfassten Koordinaten des Bezugspunktes des Instruments in ein den Bilddaten des Patienten zugrundeliegendes Koordinatensystem transformieren zu können. Auf Basis dieser Daten kann eine lagegetreue Abbildung des medizinischen Instruments zusammen mit den vorhandenen präoperativen und/oder intraoperativen Bilddaten auf dem Monitor dargestellt werden.

Derartige Lageerfassungssysteme können Lokalisatoren umfassen, die beispielsweise optisch mit Ultraschall oder elektromagnetisch arbeiten. Bekannt sind beispielsweise auf Basis elektromagnetischer Induktion arbeitende Lageerfassungssysteme, die einen Feldgenerator aufweisen, der neben dem Patienten angeordnet ist und im Arbeitsgebiet ein i.d.R. alternierendes elektromagnetisches Wechselfeld erzeugt. An einem im Arbeitsgebiet zu navigierenden chirurgischen Instrument ist ein Lokalisator angeordnet, der mehrere Sensorspulen mit bekannter relativer Lage zueinander aufweist. Das elektromagnetische Wechselfeld induziert in diesen Sensorspulen in Abhängigkeit von der Ausrichtung der jeweiligen Sensorspule zum elektromagnetischen Wechselfeld elektrische Ströme, die charakteristisch für Ausrichtung und Position der jeweiligen Spule im elektromagnetischen Wechselfeld sind. Eine Auswerteeinheit misst die induzierten Ströme und ermittelt unter Berücksichtigung der bekannten Lage der Spulen zueinander somit die Lage der Sensorspulen im elektromagnetischen Wechselfeld. In einem Registrier- oder Kalibriervorgang ist zunächst die Instrumentenspitze des chirurgischen Instruments mit einer vorbestimmten Ausrichtung an einen in dem Lageerfassungssystem definierten Referenzpunkt heranzuführen und die Lage des Lokalisators bei Erreichen des Referenzpunkts zu bestimmen. Somit ist die Lage eines mit den Sensorspulen ausgestatteten chirurgischen Instruments in dem Arbeitsgebiet durch das Lageerfassungssystem bestimmbar.

Obige Lageerfassungssysteme haben den Nachteil, dass sie im Wesentlichen nur für die Erfassung der Lage passiver chirurgischer Instrumente, wie z.B. Skalpelle oder Scheren geeignet sind. Aktive chirurgische Instrumente, wie z.B. Bohrer, Kauter oder oszillierende Sägen, senden ein elektromagnetisches Feld aus, das das elektromagnetische Wechselfeld des Feldgenerators überlagert und sich die hieraus resultierenden Interferenzen direkt auf den in der Sensorspule induzierten Strom auswirken können. Dies führt zu einer Verfälschung der von der Auswerteeinheit ermittelten Lagedaten der Sensorspule und somit des chirurgischen Instruments. Des Weiteren bergen aktive chirurgische Instrumente in einem aktiven Betriebszustand ein erhöhtes Risiko der Beschädigung benachbarten Gewebes durch den aktiven Instrumententeil.

Aus WO 02/076302 ist ein Verfahren und Gerätesystem zum Gewebeabtrag und zur Gewebebearbeitung bekannt, wobei die Lage eines aktiven chirurgischen Instruments im Operationsgebiet über eine optische Lagebestimmungseinrichtung erfasst und auf Basis der somit bestimmbaren Lageänderung des aktiven chirurgischen Instruments der Gewebeabtrag sowie die Gewebeabtragsrate im Einsatzgebiet bestimmt werden. Aufgrund dieser Daten ist der Operationsfortschritt ermittelbar und der Betriebszustand des aktiven chirurgischen Instruments steuerbar. Dabei kann die Leistung des aktiven chirurgischen Instruments bei bevorstehendem Erreichen des Operationsziels, also dem erfolgten Abtrag eines vorgegebenen Gewebevolumens, gedrosselt und bei Erreichen des Operationsziels das aktive chirurgische Instrument abgeschaltet werden.

Nachteilig an diesem Verfahren und Gerätesystem ist, dass ausschließlich optische Lageerfassungsmittel zum Einsatz kommen, die für viele chirurgische Eingriffe wegen eines eingeschränkten Sichtfelds sowie einer starken Verschmutzung der Sensoren, z.B. durch Blut, im Operationsgebiet nicht geeignet sind. Darüber hinaus berücksichtigt die Regelung des Betriebszustands des aktiven chirurgischen Instruments lediglich den Operationsfortschritt, sensible Strukturen, die potenziell durch den Operateur beschädigt werden können, werden nicht berücksichtigt.

WO 2012/109760 A1 offenbart einen chirurgischen Bohrer mit einem integrierten Navigations- und Führungssystem. Ein Verfahren zum Minimieren einer durch einen Motor bewirkten Trackinginterferenz ist aus der US 2008/0287802 A1 bekannt.

Der vorliegenden Erfindung liegt somit die Aufgabe zugrunde, ein Verfahren sowie ein medizinisches System zum Erfassen von Position und Betriebszustand von aktiven chirurgischen Instrumenten in einem Arbeitsgebiet bereitzustellen, die eine präzisere Ermittlung der Position des aktiven Instruments im Arbeitsgebiet als durch den Stand der Technik bekannt ermöglichen und das Risiko der unbeabsichtigten Beschädigung von im Arbeitsgebiet angeordneten Objekten durch das aktive chirurgische Instrument, insbesondere in einem aktiven Betriebszustand des aktiven chirurgischen Instruments, zu reduzieren.

Erfindungsgemäß wird diese Aufgabe durch das Verfahren laut Anspruch 1 und durch das System laut Anspruch 6 gelöst.

Anstelle explizit Position und Betriebszustand des aktiven chirurgischen Instruments zu ermitteln, kann auch die Stärke oder Veränderung des elektromagnetischen Feldes anzeigende Ausgangssignal des wenigsten einen ersten Sensors direkt genutzt werden, um z.B. den Betriebszustand des aktiven chirurgischen Instruments zu steuern, denn das Ausgangsignal enthält immer Information über eine relative Nähe zwischen Instrument und erstem Sensor und somit auch immer implizit Positionsdaten.

Es können also ein oder mehrere erste Sensoren vorgesehen sein. Der erste Sensor kann also entweder ein von dem aktiven chirurgischen Instrument ausgehendes elektromagnetisches Feld direkt oder eine durch den aktiven Betriebszustand des aktiven chirurgischen Instruments bewirkte Veränderung eines umgebenden elektromagnetischen Feldes erfassen sowie ggf. auch beides. Es ist zweckmäßig, durch eine Referenzmessung die Abhängigkeit des vom aktiven chirurgischen Instrument emittierten oder veränderten elektromagnetischen Felds vom Betriebszustand des aktiven chirurgischen Instruments zu bestimmen. Hierfür werden Feldparameter wie z.B. Stärke und Frequenz des elektromagnetischen Felds in Abhängigkeit vom Betriebszustand berücksichtigt. Aus den Messdaten der Referenzmessung ist ein Kennlinienfeld des aktiven chirurgischen Instruments erzeugbar. Dieses Kennlinienfeld ist bei der Bestimmung der Position sowie des Betriebszustands des aktiven chirurgischen Instruments entsprechend zu berücksichtigen.

Vorzugsweise hat das von einem jeweiligen ersten Sensor erzeugte Ausgangssignal einen Signalwert oder Pegel, der eine durch den jeweiligen ersten Sensor erfasste Stärke oder den Grad der Veränderung des elektromagnetischen Felds des aktiven chirurgischen Instruments anzeigt. Hierdurch können aus dem Signalwert oder Pegel des Ausgangssignals Rückschlüsse auf die Stärke oder die Veränderung des erfassten elektromagnetischen Felds am Sensor gezogen werden. Es ist somit ein quantitatives Widerspiegeln der Stärke oder der Veränderung des erfassten elektromagnetischen Signals möglich. Bei bekannter Position des ersten Sensors, Ausprägung des elektromagnetischen Felds sowie Verhältnis zwischen erfasster Stärke oder Veränderung des elektromagnetischen Felds zur Stärke des Ausgangssignals des ersten Sensors ist demnach anhand des Signalwerts oder Pegels des Ausgangssignals des ersten Sensors die Position des chirurgischen Instruments im Arbeitsbereich ermittelbar. Mit mehreren ersten Sensoren können genauere Positionsdaten z.B. nach Art einer Triangulation erzeugt werden.

Der Pegel des von dem jeweiligen ersten Sensor erzeugten, die erfasste Stärke oder Veränderung des elektromagnetischen Felds des aktiven chirurgischen Instruments widerspiegelnden Ausgangssignals kann auf verschiedene vorteilhafte Weise genutzt werden, z.B. zur automatischen Beeinflussung des aktiven chirurgischen Instruments oder aber auch zum Erzeugen eines von einem Arzt wahrzunehmenden Signals, dass dem Arzt die Annäherung des aktiven chirurgischen Instruments an ein Zielgebiet zur Kenntnis bringt. Ein solches wahrnehmbares Signal kann z.B. optisch oder akustisch sein, z.B. ein Ton, dessen Frequenz und/oder Modulation mit (ggf. zu starker) Annährung an eine Zielstruktur ändert oder durch optische Hervorheben kritischer Strukturen in einer präoperativ oder intraoperativ gewonnen Computertomografie. Besonders bevorzugt werden unterschiedliche Betriebszustände des aktiven chirurgischen Instruments erfasst und bei der Ermittlung der Position des aktiven chirurgischen Instruments berücksichtigt.

Die automatische Beeinflussung des aktiven medizinischen Instruments oder das erzeugte wahrnehmbare Signal können außer von der erfassten Stärke oder Veränderung des elektromagnetischen Felds auch vom Aktivitätszustand des aktiven medizinischen Instruments abhängen. Im Falle eines akustischen Signals kann die Frequenz beispielsweise die erfasste Stärke des elektromagnetischen Felds anzeigen während eine Modulation (z.B. ununterbrochener Ton vs. unterbrochener Ton) gleichzeitig den Aktivitätszustand des aktiven medizinischen Instruments anzeigen kann.

Insbesondere kann ein Warnsignal bei zu starker Annäherung an eine Zielstruktur in Abhängigkeit des Aktivitätszustands des aktiven medizinischen Instruments generiert werden.

Es ist bevorzugt, dass der Betriebszustand des aktiven chirurgischen Instruments in Abhängigkeit vom erzeugten Ausgangssignal gesteuert wird.

Weiter bevorzugt wird das aktive chirurgische Instrument gedrosselt, wenn der erste Sensor eine Stärke oder Veränderung des elektromagnetischen Felds erfasst, die einen gewählten ersten Schwellwert übersteigt. Die Drosselung kann z.B. als Funktion vom der erfassten Stärke des elektromagnetischen Felds erfolgen. Somit wird gewährleistet, dass der Betriebszustand des aktiven chirurgischen Instruments in Abhängigkeit von der Position des aktiven chirurgischen Instruments im Arbeitsbereich steuerbar ist. Des Weiteren kann eine unnötige Leistungsaufnahme des aktiven chirurgischen Instruments reduziert und ein unbeabsichtigtes Beschädigen von Objekten im Arbeitsbereich durch das aktive chirurgische Instrument verhindert werden.

Zeigt das die Stärke oder Veränderung des elektromagnetischen Felds widerspiegelnde Ausgangssignal des ersten Sensors beispielsweise eine Annäherung an ggf. sensible Zielstukturen an, kann das aktive chirurgische Instrument auch so gesteuert werden, dass es eine haptisch wahrnehmbare Rückmeldung gibt, z.B. ein intermittierender betrieb des aktiven chirurgischen Instruments der durch eine Arzt wahrgenommen werden kann, weil das Gerät "ruckelt"
Besonders bevorzugt wird das aktive chirurgische Instrument abgeschaltet, wenn der erste Sensor eine Stärke oder Veränderung des elektromagnetischen Felds erfasst, die größer als ein gewählter zweiter Schwellwert ist. Der zweite Schwellwert ist größer als der erste Schwellwert ist. Durch diese Maßnahme kann verhindert werden, dass das aktive chirurgische Instrument bei einer ungewollten Kollision mit einem Objekt im Arbeitsbereich einen aktiven Betriebszustand aufweist und das Objekt hierdurch beschädigt wird.

Vorzugsweise wird eine Bildwiederholfrequenz einer Anzeigeeinheit in Abhängigkeit von dem ermittelten Betriebszustand des aktiven chirurgischen Instruments eingestellt. Auf diese Weise ist z.B. die Ausprägung der Schneidkanten des aktiven chirurgischen Instruments für eine Bedienperson besser darstellbar.

Besonders bevorzugt entspricht eine Drehzahl einer Arbeitsspitze des aktiven chirurgischen Instruments einem ganzzahligen Vielfachen der eingestellten Bildwiederholfrequenz. Bei einer Veränderung der Drehzahl der Arbeitsspitze des aktiven chirurgischen Instruments wird die Bildwiederholfrequenz der Anzeige entsprechend angepasst, um stets einem ganzzahligen Vielfachen der Drehzahl der Arbeitsspitze des aktiven chirurgischen Instruments zu entsprechen.

Erfindungsgemäß werden die Lagedaten des aktiven chirurgischen Instruments mittels eines Instrumentensensors (also eines Lokalisators am Instrument), der am aktiven chirurgischen Instrument angeordnet ist, von einer Lageerfassungseinheit erfasst. Die Lage umfasst sowohl die Position im Raum als auch die Ausrichtung. Hierfür kann der Instrumentensensor z.B. Sensorspulen und die Lagebestimmungseinheit einen Feldgenerator umfassen, der im Arbeitsbereich ein alternierendes elektromagnetisches Wechselfeld erzeugt. Dies hat den Vorteil, dass die Lage des aktiven chirurgischen Instruments im Arbeitsbereich auch dann bestimmbar ist, wenn der Betriebszustand des aktiven chirurgischen Instruments inaktiv ist.

Es ist bevorzugt, dass die mittels des ersten Sensors erfassten Positionsdaten mit den mittels des Instrumentensensors erfassten Lagedaten des aktiven chirurgischen Instruments verglichen werden und/oder aus zusammengehörenden Positions-Lagedatenpaaren ein Positionskorrekturwert gebildet wird. Somit können z.B. Interferenzen des elektromagnetischen Felds des aktiven chirurgischen Instruments mit einem elektromagnetischen Wechselfeld der Lagebestimmungseinheit, die zu Fehlern bei der Lagebestimmung führen können, korrigiert werden. Durch diese Maßnahme kann die Genauigkeit der Lagebestimmung des aktiven chirurgischen Instruments verbessert werden.

Weil ein von einem aktiven medizinischen Gerät ausgehendes elektromagnetisches Feld eine genaue Erfassung der Position des Instrumentes mittels eines Instrumentensensors beeinträchtigen kann, können zur genaueren Lageerfassung mehrere erste Sensoren vorgesehen sein, die eine Lageerfassung z.B. auf dem Wege der Triangulation erlauben. Alternativ oder zusätzlich kann das aktive medizinische Instrument auch mit einem oder mehreren Ultraschallsensoren ausgestattet sein. Mit Hilfe eines Ultraschallsensors können auf an sich bekannte Weise Dichteänderungen im Gewebe bildartig erfasst werden. Der Vorteil ist, dass solche mittels Ultraschall gewonnen Abbildungen nicht durch elektromagnetische Felder gestört werden.

Vorzugsweise wird der Betriebszustand des aktiven chirurgischen Instruments vom aktiven chirurgischen Instrument und/oder einer Steuereinheit des aktiven chirurgischen Instruments an die Positionserfassungseinheit und/oder Lageerfassungseinheit übermittelt. Aufgrund der somit gewonnenen Parameter des Betriebszustands des aktiven chirurgischen Instruments kann die Positionserfassungseinheit und/oder Lageerfassungseinheit mit dem vom ersten Sensor erzeugte Ausgangssignal das Kennlinienfeld des aktiven chirurgischen Instruments überprüfen und bei einer vordefinierbaren Abweichung eine neue Kalibrierung des aktiven chirurgischen Instruments durchführen.

Darüber hinaus wird obige Aufgabe durch ein medizinisches System zum Lokalisieren von aktiven chirurgischen Instrumenten in einem Arbeitsbereich, mit mindestens einem aktiven chirurgischen Instrument gelöst, wobei das aktive chirurgische Instrument ausgebildet ist, im aktiven Betriebszustand ein elektromagnetisches Feld zu emittieren. Das medizinische System weist eine Positionserfassungseinheit zur Ermittlung der Position des aktiven chirurgischen Instruments im Arbeitsbereich sowie mindestens einen ersten Sensor auf. Der erste Sensor ist an einer bekannten Position im Arbeitsbereich anordenbar und ausgebildet, das elektromagnetische Feld des aktiven chirurgischen Instruments zu erfassen und ein die erfassten Parameter des elektromagnetischen Felds anzeigendes Ausgangssignal an die Positionserfassungseinheit zu übermitteln, wobei die Positionserfassungseinheit ausgebildet ist, aufgrund des Ausgangssignals und der Position des ersten Sensors die Position des aktiven chirurgischen Instruments zu ermitteln.

Vorzugsweise weist das medizinische System ein Lageerfassungssystem mit einer Lageerfassungseinheit, einem Feldgenerator und einem Instrumentensensor auf, wobei der Instrumentensensor am aktiven chirurgischen Instrument angeordnet oder anordenbar und ausgebildet ist, ein vom Feldgenerator erzeugtes elektromagnetisches Wechselfeld zu erfassen und ein das erfasste elektromagnetische Wechselfeld anzeigendes Signal an die Lageerfassungseinheit zu übermitteln. Die Lageerfassungseinheit ist ausgebildet, Lagedaten des aktiven chirurgischen Instruments aufgrund des erfassten Signals und des bekannten vom Feldgenerator erzeugten elektromagnetischen Wechselfelds zu ermitteln.

Bevorzugt ist die Positionserfassungseinheit und/oder Lageerfassungseinheit eingerichtet ist, die Positionsdaten mit den Lagedaten des aktiven chirurgischen Instruments zu vergleichen und daraus eine korrigierte Position und/oder Lage des aktiven chirurgischen Instruments zu ermitteln. Somit kann eine durch Interferenz des elektromagnetischen Felds des aktiven chirurgischen Instruments mit dem elektromagnetischen Wechselfeld des Feldgenerators falsch bestimmte Lage des aktiven chirurgischen Instruments korrigiert werden.

Vorzugsweise weist das medizinische System eine Anzeigeeinheit zur lagegetreuen Darstellung wenigstens eines von der Lageerfassungseinheit im Arbeitsgebiet erfassten aktiven chirurgischen Instruments sowie gewonnener Bilddaten eines im Arbeitsgebiet angeordneten Objekts auf. Die Bilddaten können sowohl Einzelbilder, wie z.B. Röntgenbilder oder CT-Aufnahmen, als auch Videobilder umfassen, wobei das medizinische System vorzugsweise dazu ausgebildet ist, Videobilder aufzunehmen und möglichst ohne oder mit nur geringer Verzögerung darzustellen.

In einer vorteilhaften Ausführungsform der Erfindung weist das medizinische System eine Datenschnittstelle zur Übermittlung des Betriebszustands des aktiven chirurgischen Instruments vom aktiven chirurgischen Instrument und/oder einer Instrumentensteuereinheit des aktiven chirurgischen Instruments an die Lageerfassungseinheit und/oder Positionserfassungseinheit auf. Die Datenschnittstelle kann zur kabelgebundenen oder kabellosen Übertragung, wie z.B. per Funk oder Optik, des Betriebszustands des aktiven chirurgischen Instruments ausgebildet sein.

Vorzugsweise weist das aktive chirurgische Instrument einen Elektromotor auf, der in Abhängigkeit vom Betriebszustand des aktiven chirurgischen Instruments ein Störsignal emittiert. Herkömmliche Elektromotoren weisen einen Magnete und Spulen auf und beruhen auf dem Prinzip der Induktion. Daher weisen sie bei Betrieb ein elektromagnetisches Feld auf, das durch Sensoren erfassbar ist.

Die Erfindung soll im Folgenden anhand eines Ausführungsbeispiels mit Bezug auf eine Zeichnung näher erläutert werden. Hierbei zeigt:

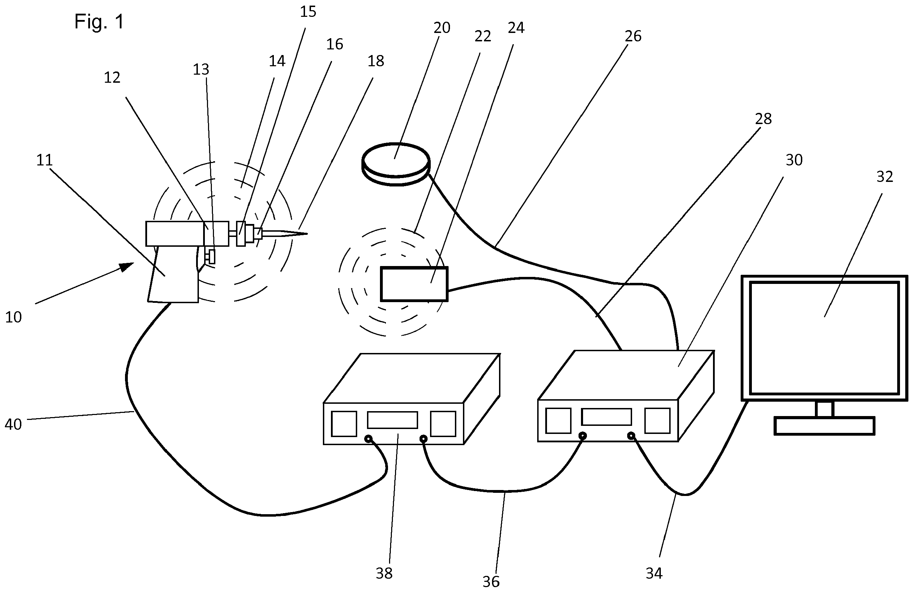

Fig. 1 eine schematische, nicht zwangsläufig maßstabsgetreue Ansicht eines erfindungsgemäßen medizinischen Systems zum Lokalisieren sowie zum Bestimmen der Betriebszustands von aktiven chirurgischen Instrumenten.

Ein erfindungsgemäßes medizinisches System, das zur Positionsbestimmung eines aktiven chirurgischen Instruments ausgebildet ist, umfasst mindestens eine Positionserfassungseinheit und einen ersten Sensor 20. Mit diesem medizinischen System ist ein medizinisches System zur Lagebestimmung eines aktiven chirurgischen Instruments 10 in einem Arbeitsbereich z.B. erweiterbar. Das derartiges medizinische System zur Lagebestimmung eines aktiven chirurgischen Instruments umfasst eine Lageerfassungseinheit, einen Feldgenerator 24 zum Emittieren eines elektromagnetischen Wechselfelds 22 in einem Arbeitsbereich, einen Instrumentensensor 16 sowie eine Anzeigeeinheit 32 zur lagegetreuen Darstellung des aktiven chirurgischen Instruments 10. Das aktive chirurgische Instrument 10 emittiert im aktiven Betriebszustand ein elektromagnetisches Feld 14.

Das medizinische System gemäß dem in Fig. 1 dargestellten Ausführungsbeispiel umfasst ein aktives chirurgisches Instrument 10, welches als chirurgischer Bohrer ausgebildet ist. Das aktive chirurgische Instrument 10 weist einen Griff 11 und einen an dem Griff 11 angeordneten Stellglied 13 zum Einstellen der Drehzahl einer Instrumentenspitze 18 auf. Zum Antreiben der Instrumentenspitze 18 weist das aktive chirurgische Instrument 10 einen in dieser Darstellung verdeckten Elektromotor 12 auf, wobei der Elektromotor 12 in der Nähe der Instrumentenspitze 18 angeordnet ist und im Betrieb das elektromagnetische Feld 14 emittiert. Die Instrumentenspitze 18 ist über eine Spanneinrichtung 15 an dem aktiven chirurgischen Instrument lösbar gehalten. Somit kann die Instrumentenspitze 18 z.B. aufgrund von Verschleiß oder Verunreinigung leicht demontiert und durch eine neue Instrumentenspitze 18 ersetzt oder wieder in einen Zustand versetzt werden, der den Anforderungen an die Instrumentenspitze 18 gerecht wird.

Der Elektromotor 12 emittiert im Betrieb ein elektromagnetisches Feld 14, das in der Abbildung durch konzentrische Kreissegmente symbolisch dargestellt ist. Das elektromagnetische Feld 14 breitet sich im Wesentlichen kugelförmig vom Elektromotor 12 aus und weist eine Feldfrequenz und eine Feldstärke auf. Das aktive chirurgische Instrument 10 ist über ein Instrumentenkabel 40 mit einer Instrumentensteuereinheit 38 verbunden. Die Instrumentensteuereinheit 38 versorgt das aktive chirurgische Instrument 10 über das Instrumentenkabel 40 mit Strom.

Ein erster Sensor 20 zum Erfassen des elektromagnetischen Felds 14 ist über eine Sensorleitung 26 mit einer Navigationseinheit 30 verbunden. Die Navigationseinheit 30 umfasst in dieser Ausführungsform eine Positionserfassungseinheit und eine Lageerfassungseinheit. Alternativ können Positionserfassungseinheit und Lageerfassungseinheit auch als separate Module ausgebildet sein, so dass z.B. ein medizinisches System, das bereits eine Lageerfassungseinheit umfasst, ohne großen Aufwand um eine Positionserfassungseinheit mit einem ersten Sensor ergänzbar ist.

Der erste Sensor 20 ist in einem Arbeitsgebiet z.B. in der Nähe eines nicht dargestellten Objekts anordenbar, wobei das Objekt durch die Instrumentenspitze 18, insbesondere im aktiven Betriebszustand des aktiven chirurgischen Instruments, potenziell beschädigbar ist. Bei Annäherung des aktiven chirurgischen Instruments 10 im aktiven Betriebszustand an den ersten Sensor 20 induziert das elektromagnetische Feld 14 einen Strom im ersten Sensor 20, der als Ausgangssignal über eine Sensorleitung 26 an die Navigationseinheit 30 weitergeleitet wird. Da die Lagedaten des ersten Sensors 20 im Arbeitsbereich bekannt sind, kann die Navigationseinheit 30 aufgrund der Ausprägung des empfangenen Ausgangssignals des ersten Sensors 20 die Position des Elektromotors 12 und somit auch die Position des aktiven chirurgischen Instruments 10 im Arbeitsbereich bestimmen. Wird kein Signal vom ersten Sensor 20 empfangen, so bedeutet dies entweder, dass das aktive chirurgische Instrument 10 zu weit vom ersten Sensor 20 entfernt oder im passiven Betriebszustand, also ausgeschaltet ist.

Zur Visualisierung der bestimmten Positionsdaten des aktiven chirurgischen Instruments 10 ist die Navigationseinheit 30 über ein Monitorkabel 34 mit einer Anzeigeeinheit 32 verbunden. Somit kann z.B. die Instrumentenspitze 18 auf der Anzeigeeinheit dargestellt werden. Zweckmäßigerweise werden diese Bilddaten mit weiteren Bilddaten des Operationsgebiets des Patienten, die entweder präoperativ oder intraoperativ gewonnen werden, wie z.B. CT-, Röntgen- oder Videobilddaten, auf der Anzeigeeinheit 32 überlagert dargestellt.

Die Navigationseinheit 30 ist über ein Generatorkabel 28 mit einem Feldgenerator 24 verbunden, der ein in der Abbildung mittels konzentrischer Kreissegmente schematisch dargestelltes elektromagnetisches Wechselfeld 22 erzeugt. Dieses elektromagnetische Wechselfeld 22 ist von einem in der Nähe der Instrumentenspitze 18 am aktiven chirurgischen Instrument 10 angeordneten Instrumentensensor 16 erfassbar. Durch das elektromagnetische Wechselfeld 22 wird abhängig von der Lage des Instrumentensensors 16 im Instrumentensensor 16 ein Strom induziert, der über das Instrumentenkabel 40 an die Instrumentensteuereinheit 38 weitergeleitet wird. Über ein Verbindungskabel 36 wird dieser induzierte Strom an die Navigationseinheit 30 weitergeleitet und dort ausgewertet, um die Lage des aktiven chirurgischen Instruments 10 relativ zum Patienten zu bestimmen und auf der Anzeigeeinheit 32 darzustellen. Diese zusätzliche Möglichkeit der Lagebestimmung hat den Vorteil, dass die Lage des aktiven chirurgischen Instruments 10 relativ zum Patienten auch bestimmbar ist, wenn das aktive chirurgische Instrument 10 inaktiv ist und der Elektromotor 12 kein elektromagnetisches Feld 14 erzeugt. Das System kann derart konfiguriert werden, dass der Feldgenerator 24 inaktiv ist oder nur pulsierend betrieben wird, wenn das aktive chirurgische Instrument 10 aktiv ist und der Elektromotor 12 ein elektromagnetisches Feld 14 erzeugt, um etwaige Messstörungen durch Interferenzen zwischen dem elektromagnetischen Feld 14 des Elektromotors 12 und dem elektromagnetischen Wechselfeld 22 des Feldgenerators 24 zu verhindern oder auszugleichen.

Wenn die Positionserfassungseinheit bzw. Navigationseinheit 30 feststellt, dass das aktive chirurgische Instrument 10 oder die Instrumentenspitze 18 einen ersten Mindestabstand zu einem in dieser Darstellung nicht abgebildeten Objekt im Arbeitsgebiet unterschreitet, kann von der Positionserfassungseinheit bzw. Navigationseinheit 30 ein Steuersignal an die Instrumentensteuereinheit 38 übermittelt werden, um die Leistung des aktiven chirurgischen Instruments 10 zu drosseln. Diese Drosselung kann beispielsweise sprungartig oder kontinuierlich erfolgen. Die Drosselung kann auch direkt am aktiven chirurgischen Instrument 10 erfolgen, ohne ein Steuersignal an die Instrumentensteuereinheit 38 zu senden. Hierfür könnte z.B. ein vorzugsweise regelbarer Widerstand am Elektromotor 12 angeordnet sein.

Alternativ oder zusätzlich kann eine Warnung an den Bediener, z.B. ein akustisches oder optisches Signal ausgegeben werden. Als optisches Signal kann beispielsweise ein Farbwechsel auf der Anzeigeeinheit 32 erfolgen. Der Farbwechsel kann sich auch über ein Spektrum erstrecken und in Abhängigkeit vom Abstand der Instrumentenspitze 18 des aktiven chirurgischen Instruments 10 von dem Objekt im Arbeitsgebiet erfolgen. Hierdurch kann der Bediener gewarnt werden, dass eine Kollision mit einem Objekt im Arbeitsbereich potenziell bevorsteht und das aktive chirurgische Instrument 10 von diesem Objekt wieder entfernt werden sollte. Durch die Drosselung des aktiven chirurgischen Instruments 10 wird eine Beschädigung des Objekts durch das aktive chirurgische Instrument bei einer Kollision weiter reduziert. Ferner ist die Drosselung auch ein fühlbarer und/oder ggf. hörbarer und/oder sichtbarer Hinweis für den Bediener, dass das aktive chirurgische Instrument 10 einen ersten Mindestabstand zu einem bestimmten Objekt im Arbeitsbereich unterschritten hat.

Stellt die Positionserfassungseinheit bzw. Navigationseinheit 30 weiter fest, dass das aktive chirurgische Instrument 10 einen zweiten Mindestabstand zu dem Objekt im Arbeitsbereich unterschreitet, wobei der zweite Mindestabstand vorzugsweise kleiner als der erste Mindestabstand ist, kann von der Positionserfassungseinheit bzw. Navigationseinheit 30 ein weiteres Steuersignal an die Instrumentensteuereinheit 38 übermittelt werden, um das aktive chirurgische Instrument 10 zu deaktivieren. Alternativ kann die Abschaltung des aktiven chirurgischen Instruments 10 direkt am aktiven chirurgischen Instrument 10, z.B. durch Trennung der Stromversorgung am Elektromotor 12 und ohne Übermittlung eines Steuersignals an die Instrumentensteuereinheit 38 erfolgen. Hierdurch wird das Risiko der Beschädigung eines im Arbeitsbereich angeordneten Objekts durch ein aktives chirurgisches Instrument 10 bei einer Kollision mit einer sensiblen Struktur des Patienten verringert, da von dem aktiven chirurgischen Instrument 10 im deaktivierten Betriebszustand ein geringeres Gefahrenpotenzial als im aktiven Betriebszustand ausgeht. Darüber hinaus wird der Bediener durch den Stillstand des aktiven chirurgischen Instruments 10 dazu veranlasst, das aktive chirurgische Instrument 10 von dem Objekt im Arbeitsbereich weg zu navigieren.

Dieses Ausführungsbeispiel soll lediglich schematisch den Gegenstand der vorliegenden Erfindung darstellen und die Erfindung nicht auf diese konkrete Ausführungsform einschränken. Es sind Konfigurationen im Rahmen der Erfindung vorgesehen, bei denen Navigationseinheit 30 und Instrumentensteuereinheit 38 eine gemeinsame Einheit bilden. Kabelverbindungen können ggf. auch durch Funkverbindungen ersetzt werden. Die Anzahl der verwendeten Sensoren ist zweckmäßigerweise größer als in dem dargestellten Ausführungsbeispiel, um die Genauigkeit des Navigationssystems zu verbessern. Ein erster Sensor kann z.B. eine Sensoreinheit sein und kann ebenfalls eine Gruppe von ersten Sensoren umfassen. Des Weiteren können Mittel vorgesehen sein, die die Ausprägung des elektromagnetischen Wechselfelds 22 des Feldgenerators 24 in Abhängigkeit von der Ausprägung des elektromagnetischen Störfelds 14 des Elektromotors 12 regeln.

### Bezugszeichen:

- 10: aktives chirurgisches Instrument
- 11: Griff
- 12: Elektromotor
- 13: Stellglied
- 14: elektromagnetisches Feld
- 15: Spanneinrichtung
- 16: Instrumentensensor
- 18: Instrumentenspitze
- 20: erster Sensor
- 22: elektromagnetisches Wechselfeld
- 24: Feldgenerator
- 26: Sensorleitung
- 28: Generatorkabel
- 30: Navigationseinheit
- 32: Anzeigeeinheit
- 34: Monitorkabel
- 36: Verbindungskabel
- 38: Instrumentensteuereinheit
- 40: Instrumentenkabel

## Patentansprüche

1. Verfahren zum Erfassen von Position und Betriebszustand eines aktiven chirurgischen Instruments (10), wobei das aktive chirurgische Instrument (10) in einem eingeschalteten Betriebszustand mindestens ein elektromagnetisches Feld (14) emittiert, mit den Verfahrensschritten:
- Positionieren eines ersten Sensors (20) zum Erfassen des vom aktiven chirurgischen Instrument (10) in dessen aktiven Betriebszustand ausgehenden elektromagnetischen Felds (14) an einer bekannten Position;
- Erfassen des von dem aktiven chirurgischen Instrument (10) in dessen aktiven Betriebszustand ausgehenden elektromagnetischen Felds (14) mittels des ersten Sensors (20);
- Erzeugen eines Ausgangssignals durch den ersten Sensor (20), wobei das Ausgangssignal das Erfassen des von dem aktiven chirurgischen Instrument (10) ausgehenden elektromagnetischen Felds (14) durch den ersten Sensor (20) anzeigt; und
- Ermitteln von Position und Betriebszustand des aktiven chirurgischen Instruments (10) auf Basis des Ausgangsignals und der bekannten Position des ersten Sensors (20) sowie erzeugen von Positionsdaten,
**dadurch gekennzeichnet, dass** Lagedaten des aktiven chirurgischen Instruments (10) mittels eines Instrumentensensors (16), der am aktiven chirurgischen Instrument (10) angeordnet ist, von einer Lageerfassungseinheit erfasst werden.

2. Verfahren nach Anspruch 1, **dadurch gekennzeichnet, dass** das vom ersten Sensor (20) erzeugte Ausgangssignal einen Signalwert oder Pegel hat, der einen durch den ersten Sensor (20) erfassten Grad einer Veränderung des elektromagnetischen Felds (14) und/oder eine durch den ersten Sensor (20) erfasste Stärke des elektromagnetischen Felds (14) des aktiven chirurgischen Instruments (10) anzeigt.

3. Verfahren nach Anspruch 1 oder 2,
**dadurch gekennzeichnet, dass** unterschiedliche Betriebszustände des aktiven chirurgischen Instruments (10) erfasst und bei der Ermittlung der Position des aktiven chirurgischen Instruments (10) berücksichtigt werden.

4. Verfahren nach einem der vorangehenden Ansprüche,
**dadurch gekennzeichnet, dass** eine Bildwiederholfrequenz einer Anzeigeeinheit (32) in Abhängigkeit von dem ermittelten Betriebszustand des aktiven chirurgischen Instruments (10) eingestellt wird.

5. Verfahren nach einem der vorstehenden Ansprüche,
**dadurch gekennzeichnet, dass** die mittels des ersten Sensors (20) erfassten Positionsdaten mit den mittels des Instrumentensensors (16) erfassten Lagedaten des aktiven chirurgischen Instruments (10) verglichen werden und/oder aus zusammengehörenden Positions-Lagedatenpaaren ein Positionskorrekturwert gebildet wird.

6. Medizinisches System zum Lokalisieren und zur Bestimmung des Betriebszustands von aktiven chirurgischen Instrumenten (10) in einem Arbeitsbereich, mit mindestens einem aktiven chirurgischen Instrument (10), wobei das aktive chirurgische Instrument (10) ausgebildet ist, im aktiven Betriebszustand ein elektromagnetisches Feld (14) zu emittieren,
wobei das medizinische System eine Positionserfassungseinheit zur Ermittlung der Position des aktiven chirurgischen Instruments im Arbeitsbereich sowie mindestens einen ersten Sensor (20) aufweist, wobei der erste Sensor (20) an einer bekannten Position im Arbeitsbereich anordenbar ist, wobei der erste Sensor (20) ausgebildet ist, ein von dem aktiven chirurgischen Instrument (10) ausgehendes oder verändertes elektromagnetisches Feld (14) zu erfassen und wenigstens ein einen erfassten Parameter des elektromagnetischen Felds (14) anzeigendes Ausgangssignal an die Positionserfassungseinheit zu übermitteln, wobei die Positionserfassungseinheit ausgebildet ist, aufgrund des Ausgangssignals und der Position des ersten Sensors (20) die Position und den Betriebszustand des aktiven chirurgischen Instruments (10) zu ermitteln und entsprechende Positionsdaten zu erzeugen, **gekennzeichnet durch** ein Lageerfassungssystem mit einer Lageerfassungseinheit, einem Feldgenerator und einem Instrumentensensor (16), wobei der Instrumentensensor (16) am aktiven chirurgischen Instrument (10) anordenbar ist, wobei der Instrumentensensor (16) ausgebildet ist, ein vom Feldgenerator erzeugtes elektromagnetisches Wechselfeld (22) zu erfassen und ein das erfasste elektromagnetische Wechselfeld (22) anzeigendes Signal an die Lageerfassungseinheit zu übermitteln, wobei die Lageerfassungseinheit ausgebildet ist, Lagedaten des aktiven chirurgischen Instruments (10) aufgrund des erfassten Signals und des bekannten vom Feldgenerator erzeugten elektromagnetischen Wechselfelds (22) zu ermitteln.

7. Medizinisches System nach Anspruch 6,
**dadurch gekennzeichnet, dass** die Positionserfassungseinheit und/oder Lageerfassungseinheit eingerichtet ist, die Positionsdaten mit den Lagedaten des aktiven chirurgischen Instruments (10) zu vergleichen und daraus eine korrigierte Position und/oder Lage des aktiven chirurgischen Instruments (10) zu ermitteln.

8. Medizinisches System nach Anspruch 6 oder 7,
**gekennzeichnet durch** eine Anzeigeeinheit (32) zur lagegetreuen Darstellung der von der Lageerfassungseinheit im Arbeitsgebiet erfassten aktiven chirurgischen Instrumente (10) sowie gewonnener Bilddaten eines im Arbeitsgebiet angeordneten Objekts.

9. Medizinisches System nach einem der Ansprüche 6 bis 8,
**gekennzeichnet durch** eine Datenschnittstelle zur Übermittlung des Betriebszustands des aktiven chirurgischen Instruments (10) vom aktiven chirurgischen Instrument (10) und/oder einer Instrumentensteuereinheit (38) des aktiven chirurgischen Instruments (10) an die Lageerfassungseinheit und/oder Positionserfassungseinheit.

## Claims

1. A method for detecting a location and operating state of an active surgical instrument (10), the active surgical instrument (10) emitting at least one electromagnetic field (14) in a switched-on operating state, comprising the following method steps:
- positioning a first sensor (20) for detecting the electromagnetic field (14) emanating from the active surgical instrument (10) in the active operating state thereof at a known location;
- detecting by means of the first sensor (20) the electromagnetic field (14) emanating from the active surgical instrument (10) in the active operating state thereof;
- generating an output signal by the first sensor (20), the output signal indicating the detection by the first sensor (20) of the electromagnetic field (14) emanating from the active surgical instrument (10); and
- establishing a location and operating state of the active surgical instrument (10) on the basis of the output signal and the known location of the first sensor (20), and generating location data.
**characterized in that** the position data of the active surgical instrument (10) is detected by a position detection unit by means of an instrument sensor (16) arranged at the active surgical instrument (10).

2. The method as claimed in claim 1, wherein the output signal generated by the first sensor (20) has a signal value or level which indicates a degree of change in the electromagnetic field (14) detected by the first sensor (20) and/or a strength of the electromagnetic field (14) of the active surgical instrument (10) detected by the first sensor (20).

3. The method as claimed in claim 1 or 2,
**characterized in that** different operating states of the active surgical instrument (10) are detected and taken into account when establishing the location of the active surgical instrument (10).

4. The method as claimed in one of the preceding claims,
**characterized in that** a refresh rate of a display unit (32) is set in a manner dependent on the established operating state of the active surgical instrument (10).

5. The method as claimed in one of the preceding claims,
**characterized in that** there is a comparison of the location data, detected by means of the first sensor (20), and the position data, detected by means of the instrument sensor (16), of the active surgical instrument (10) and/or a location correction value is formed from associated location-position data pairs.

6. A medical system for localizing and for determining the operating state of active surgical instruments (10) in a work region, with at least one active surgical instrument (10), the active surgical instrument (10) being embodied to emit an electromagnetic field (14) in the active operating state,
wherein the medical system has a location detection unit for establishing the location of the active surgical instrument in the work region and at least one first sensor (20), the first sensor (20) being arrangeable at a known location in the work region, the first sensor (20) being embodied to detect an electromagnetic field (14) emanating from, or changed by, the active surgical instrument (10) and to transmit at least one output signal indicating a detected parameter of the electromagnetic field (14) to the location detection unit, the location detection unit being embodied to establish the location of the active surgical instrument (10) on the basis of the output signal and the location of the first sensor (20) and the operating state and to generate corresponding location data,
**characterized by** a position detection system with a position detection unit, a field generator and an instrument sensor (16), the instrument sensor (16) being arrangeable at the active surgical instrument (10), the instrument sensor (16) being embodied to detect an alternating electromagnetic field (22) generated by the field generator and to transmit a signal indicating the detected alternating electromagnetic field (22) to the position detection unit, the position detection unit being embodied to establish position data of the active surgical instrument (10) on the basis of the detected signal and the known alternating electromagnetic field (22) generated by the field generator.

7. The medical system as claimed in claim 6,
**characterized in that** the location detection unit and/or position detection unit is configured to compare the location data and the position data of the active surgical instrument (10) and to establish a corrected location and/or position of the active surgical instrument (10) therefrom.

8. The medical system as claimed in claim 6 or 7,
**characterized in that** a display unit (32) for depicting, true to the position, the active surgical instrument (10) detected by the position detection unit in the work region and obtained image data of an object arranged in the work region.

9. The medical system as claimed in one of the claims 6 to 8,
**characterized by** a data interface for transmitting the operating state of the active surgical instrument (10) from the active surgical instrument (10) and/or an instrument control unit (38) of the active surgical instrument (10) to the position detection unit and/or location detection unit.

## Revendications

1. Procédé de détection de la position et de l'état de fonctionnement d'un instrument (10) chirurgical actif, dans lequel l'élément (10) chirurgical actif émet, dans un état de fonctionnement en service, au moins un champ (14) électromagnétique, comprenant les stades de procédé :
- mise en une position connue d'un premier capteur (20) de détection du champ (14) électromagnétique, issu de l'instrument (10) chirurgical actif dans son état de fonctionnement actif ;
- détection, au moyen du premier capteur (20), du champ (14) électromagnétique issu de l'instrument (10) chirurgical actif dans son état de fonctionnement actif ;
- production d'un signal de sortie par le premier capteur (20), le signal de sortie indiquant la détection du champ (14) électromagnétique issu de l'instrument (10) chirurgical actif par le premier capteur (20) ; et
- détermination de la position et l'état de fonctionnement de l'instrument (10) chirurgical actif sur la base du signal de sortie et de la position connue du premier capteur (20), ainsi que production de données de position,
**caractérisé en ce que** l'on détecte, par une unité de détection d'emplacement, des données d'emplacement de l'instrument (10) chirurgical actif, au moyen d'un capteur (16) d'instrument, qui est disposé sur l'instrument (10) chirurgical actif.

2. Procédé suivant la revendication 1, **caractérisé en ce que** le signal de sortie produit par le premier capteur (20) a une valeur de signal ou un niveau, qui indique un degré, détecté par le premier capteur (20), d'une variation du champ (14) électromagnétique et/ou une intensité, détectée par le premier capteur (20), du champ (14) électromagnétique de l'instrument (10) chirurgical actif.

3. Procédé suivant la revendication 1 ou 2,
**caractérisé en ce que** l'on détecte des états de fonctionnement différents de l'instrument (10) chirurgical actif et on en tient compte lorsque l'on détermine la position de l'instrument (10) chirurgical actif.

4. Procédé suivant l'une des revendications précédentes,
**caractérisé en ce que** l'on règle une fréquence de rafraichissement d'image d'une unité (32) d'affichage, en fonction de l'état de fonctionnement déterminé de l'instrument (10) chirurgical actif.

5. Procédé suivant l'une des revendications précédentes,
**caractérisé en ce que** l'on compare les données de position, détectées au moyen du premier capteur (20) aux données d'emplacement, détectées au moyen du capteur (16) d'instrument, de l'instrument (10) chirurgical actif et/ou **en ce que** l'on forme une valeur de correction de position à partir de paires de données de position-d'emplacement de capteur de position allant ensembles.

6. Système médical de localisation et de détermination de l'état de fonctionnement d'instruments (10) chirurgicaux actifs dans une région de travail, comprenant au moins un instrument (10) chirurgical actif, l'instrument (10) chirurgical actif étant constitué pour émettre, dans un état de fonctionnement actif, un champ (14) électromagnétique,
dans lequel le système médical a une unité de détection de position pour déterminer la position de l'instrument chirurgical actif dans la région de travail, ainsi qu'au moins un premier capteur (20), le premier capteur (20) pouvant être mis en une position connue dans la région de travail, le premier capteur (20) étant constitué pour détecter un champ (14) électromagnétique issu de l'instrument (10) chirurgical actif ou modifié par celui-ci et pour transmettre à l'unité de détection de position au moins un signal de sortie indiquant un paramètre détecté du champ (14) électromagnétique, l'unité de détection de position étant constituée pour déterminer, sur la base du signal de sortie et de la position du premier capteur (20), la position et l'état de fonctionnement de l'instrument (10) chirurgical actif et pour produire des données de position correspondantes,
**caractérisé par** un système de détection d'emplacement ayant une unité de détection d'emplacement, un générateur de champ et un capteur (16) d'instrument, le capteur (16) d'instrument pouvant être disposé sur l'instrument (10) chirurgical actif, le capteur (16) d'instrument étant constitué pour détecter un champ (22) alternatif électromagnétique produit par le générateur de champ et pour transmettre à l'unité de détection d'emplacement le signal indiquant le champ (22) alternatif électromagnétique détecté, l'unité de détection d'emplacement étant constituée pour déterminer des données d'emplacement de l'instrument (10) chirurgical actif sur la base du signal détecté et du champ (22) alternatif électromagnétique connu produit par le générateur de champ.

7. Système médical suivant la revendication 6,
**caractérisé en ce que** l'unité de détection de position et/ou l'unité de détection d'emplacement est conçue pour comparer les données de position aux données d'emplacement de l'instrument (10) chirurgical actif et pour en déterminer une position corrigée et/ou un emplacement de l'instrument (10) chirurgical actif.

8. Système médical suivant la revendication 6 ou 7,
**caractérisé par** une unité (32) d'affichage pour la représentation fidèle en emplacement d'instruments (10) chirurgicaux actifs détectés par l'unité de détection d'emplacement dans la région de travail, ainsi que de données d'image obtenues d'un objet disposé dans la région de travail.

9. Système médical suivant l'une des revendications 6 à 8,
**caractérisé par** une interface de données pour transmettre l'état de fonctionnement de l'instrument (10) chirurgical actif de l'instrument (10) chirurgical actif et/ou d'une unité (38) de commande d'instruments de l'instrument (10) chirurgical actif à l'unité de détection d'emplacement et/ou à l'unité de détection de position.
